# EUROPÄISCHE PATENTSCHRIFT

(11) **EP 0 638 548 B1**
(45) Veröffentlichungstag und Bekanntmachung des Hinweises auf die Patenterteilung: **22.04.1998**
(21) Anmeldenummer: 94112383.8
(22) Anmeldetag: 08.08.1994
(51) Int. Cl.: C07C 279/22, C07C 335/16, C07D 295/195

(54) **Harnstoffsubstituierte Benzoylguanidine, Verfahren zu ihrer Herstellung, ihre Verwendung als Medikament oder Diagnostikum sowie sie enthaltendes Medikament**
Urea-subsituted benzoylguanidines, process for their preparation, their use as a medicament or diagnostic agent and a medicament containing them
Benzoylguanidines substituées par uréa, procédé pour leur préparation, leur utilisation comme médicament ou agent diagnostique et médicament les contenant

(30) Priorität: 13.08.1993 DE 4327244
(43) Veröffentlichungstag der Anmeldung: 15.02.1995
(73) Patentinhaber: HOECHST AKTIENGESELLSCHAFT, 65926 Frankfurt am Main (DE)
(72) Erfinder: Schwark, Jan-Robert, Dr., D-65929 Frankfurt (DE); Lang, Hans-Jochen, Dr., D-65719 Hofheim (DE); Kleemann, Heinz-Werner, Dr., D-61350 Bad Homburg (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- EP-A- 0 556 674

## Beschreibung

Die Erfindung betrifft Benzoylguanidine der Formel I 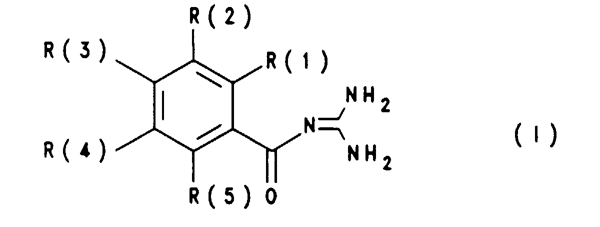 worin bedeuten:
- R(1), R(3) oder R(4): -NR(6) C =X NR(7)R(8),
X Sauerstoff, S,
R(6) Wasserstoff, (C₁-C₈)-Alkyl, (C₁-C₈)-Perfluoralkyl, (C₃-C₈)-Alkenyl, -CₙH₂ₙ-R(9),
n Null, 1, 2, 3 oder 4,
R(9) (C₃-C₈)-Cycloalkyl, Phenyl, Biphenylyl oder Naphthyl, wobei die Aromaten nicht substituiert oder substituiert sind mit 1 - 3 Substituenten aus der Gruppe F, Cl, CF₃, Methyl, Methoxy oder NR(10)R(11),
R(10) und R(11) H, (C₁-C₄)-Alkyl oder (C₁-C₄)-Perfluoralkyl;
R(7) Wasserstoff, (C₁-C₈)-Alkyl, (C₁-C₈)-Perfluoralkyl, (C₃-C₈)-Alkenyl, -CₒH₂ₒ-R(12),
o Null, 1, 2, 3 oder 4,
R(12) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(13)R(14),
R(13) und R(14) H, (C₁-C₄)-Alkyl oder (C₁-C₄)-Perfluoralkyl;
R(8) definiert wie R(7);
wobei R(7) und R(8) auch gemeinsam 4 oder 5 Methylengruppen sein können, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5) unabhängig voneinander
Wasserstoff, F, Cl, Br, I, -Oₜₐ(C₁-C₈)-Alkyl, -O_{tb}(C₃-C₈)-Alkenyl, -O_{tc}(CH₂)_{b}C_{d}F_{2d+1}, -O_{td}CₚH₂ₚR(18),
oder bis zu 2 Gruppen CN, NO₂, NR(16)R(17),
- b: Null oder 1,
- d: 1, 2, 3, 4, 5, 6 oder 7,
- ta: Null oder 1,
- tb: Null oder 1,
- tc: Null oder 1,
- td: Null oder 1,
- p: Null, 1, 2, 3 oder 4,
- R(18): (C₃-C₈)-Cycloalkyl, Phenyl, Biphenylyl oder Naphthyl, wobei die Aromaten nicht substituiert oder substituiert sind mit 1 - 3 Substituenten aus der Gruppe F, Cl, CF₃, Methyl, Methoxy oder NR(19)R(20),
R(19) und R(20) Wasserstoff, (C₁-C₄)-Alkyl, (C₁-C₄)-Perfluoralkyl,
- R(16): Wasserstoff, (C₁-C₈)-Alkyl, (C1 (C₁-C₈)-Perfluoralkyl, (C₃-C₈)-Alkenyl, -C_{q}H_{2q}-R(21),
q Null, 1, 2, 3 oder 4,
R(21) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(22)R(23),
R(22) und R(23) Wasserstoff, (C₁-C₄)-Alkyl oder (C₁-C₄)-Perfluoralkyl,
- R(17): Wasserstoff, (C₁-C₈)-Alkyl, (C₁-C₈)-Perfluoralkyl, (C₃-C₈)-Alkenyl, -CᵣH₂ᵣ-R(24),
r Null, 1, 2, 3 oder 4,
R(24) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(25)R(26),
R(25) und R(26) Wasserstoff, (C₁-C₄)-Alkyl oder (C₁-C₄)-Perfluoralkyl; wobei R(16) und R(17) auch gemeinsam 4 oder 5 Methylengruppen sein können, von denen eine CH₂-Gruppe durch Sauerstoff, S, NH, N-CH₃ oder N-Benzyl ersetzt sein kann.

Bevorzugt sind Verbindungen der Formel I, in der bedeuten:
- R(1), R(3) oder R(4): -NR(6) C =X NR(7)R(8),
X Sauerstoff, S,
R(6) Wasserstoff, (C₁-C₈)-Alkyl, (C₁-C₈)-Perfluoralkyl, (C₃-C₈)-Alkenyl, -CₙH₂ₙ-R(9),
n Null oder 1,
R(9) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(10)R(11),
R(10) und R(11) H, CH₃,
R(7) Wasserstoff, (C₁-C₈)-Alkyl, (C₁-C₈)-Perfluoralkyl, (C₃-C₈)-Alkenyl, -CₒH₂ₒ-R(12),
o Null oder 1,
R(12) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(13)R(14),
R(13) und R(14) H, CH₃,
R(8) definiert wie R(7),
wobei R(7) und R(8) auch gemeinsam 4 oder 5 Methylengruppen sein können, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5) sind unabhängig voneinander
Wasserstoff, F, Cl, Br, I, (C₁-C₈)-Alkyl, (C₃-C₈)-Alkenyl, (C₁-C₇)-perfluoralkyl, CₚH₂ₚR(18),
oder bis zu 2 Gruppen CN, NO₂, NR(16)R(17),
p Null oder 1,
R(18) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(19)R(20),
R(19) und R(20) Wasserstoff, CH₃,
R(16) Wasserstoff, (C₁-C₈)-Alkyl, (C₁-C₈)-Perfluoralkyl, (C₃-C₈)-Alkenyl, -CₚH₂ₚ-R(21),
q Null oder 1,
R(21) (C₃-C₈)-Cycloalkyl, Phenyl,
das nicht substituiert oder substituiert ist mit 1 - 3 Substituenten aus der Gruppe F, Cl, CF₃, Methyl, Methoxy oder
NR(22)R(23),
R(22) und R(23) Wasserstoff, CH₃,
R(17) Wasserstoff, (C₁-C₈)-Alkyl, (C₁-C₈)-Perfluoralkyl, (C₃-C₈)-Alkenyl, -CᵣH₂ᵣ-R(24),
r Null oder 1,
R(24) (C₃-C₈)-Cycloalkyl, Phenyl,
das nicht substituiert oder substituiert ist mit 1 - 3 Substituenten aus der Gruppe F, Cl, CF₃, Methyl, Methoxy oder
NR(25)R(26),
R(25) und R(26) Wasserstoff oder CH₃,
wobei R(16) und R(17) auch gemeinsam 4 oder 5 Methylengruppen sein können, von denen eine CH₂-Gruppe durch Sauerstoff, S, NH, N-CH₃ oder N-Benzyl ersetzt sein kann.

Besonders bevorzugt sind Verbindungen der Formel I, in der bedeuten:
- R(1), R(3) oder R(4): -NR(6) C=X NR(7)R(8),
X Sauerstoff, S,
R(6) Wasserstoff, (C₁-C₄)-Alkyl, (C₁-C₄)-Perfluoralkyl,
R(7) Wasserstoff, (C₁-C₄)-Alkyl, (C₁-C₄)-Perfluoralkyl, (C₃-C₈)-Alkenyl, -CₒH₂ₒ-R(12),
o Null oder 1,
R(12) (C₃-C₈)-Cycloalkyl, Phenyl,
das nicht substituiert oder substituiert sind mit 1 - 3 Substituenten aus der Gruppe F, Cl, CF₃, Methyl, Methoxy oder NR(13)R(14),
R(13) und R(14) H, CH₃,
R(8) definiert wie R(7),
wobei R(7) und R(8) auch gemeinsam 4 oder 5 Methylengruppen sein können, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5) sind unabhängig voneinander
Wasserstoff, F, Cl, Br, I, (C₁-C₄)-Alkyl, (C₁-C₄)-Perfluoralkyl, CₚH₂ₚR(18), oder bis zu 2 Gruppen CN, NO₂, NR(16)R(17),
- p: Null oder 1,
- R(18): (C₃-C₈)-Cycloalkyl, Phenyl,
das nicht substituiert oder substituiert ist mit 1 - 3
Substituenten aus der Gruppe F, Cl, CF₃, Methyl, Methoxy oder NR(19)R(20),
R(19) und R(20) Wasserstoff, CH₃,
- R(16): Wasserstoff, (C₁-C₄)-Alkyl, (C₁-C₄)-Perfluoralkyl,
- R(17): Wasserstoff, (C₁-C₄)-Alkyl, (C₁-C₄)-Perfluoralkyl, (C₃-C₈)-Alkenyl, -CᵣH₂ᵣ-R(24),
r Null oder 1,
R(24) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(25)R(26),
R(25) und R(26) Wasserstoff oder CH₃,
wobei R(16) und R(17) auch gemeinsam 4 oder 5 Methylengruppen sein können, von denen eine CH₂-Gruppe durch Sauerstoff, S, NH, N-CH₃ oder N-Benzyl ersetzt sein kann,
sowie deren pharmazeutisch verträgliche Salze.

Enthält einer der Substituenten R(1) bis R(5) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindung I, dadurch gekennzeichnet, daß man
eine Verbindung der Formel II 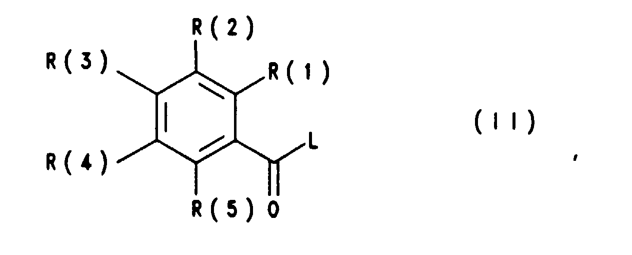 worin R(1) bis R(5) die angegebene Bedeutung besitzen und L für eine leicht nucleophil substituierbare leaving group steht, mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L=OCH₃ durch Behandeln mit gasförmigem HCI in Methanol, die Imidazolide der Formel II durch Behandeln mit Carbonyldiimidazol [L= 1-Imidazolyl, Staab, Angew. Chem. Int. Ed. Engl. 1, 351-367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit
O-[(Cyano(ethoxycarbonyl)methylen)amino]-1,1,3,3-tetramethyluroniumtetrafluorborat ("TOTU") [Proceedings of the 21. European Peptide Symposium, Peptids 1990, Editors E. Girald and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = L=OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 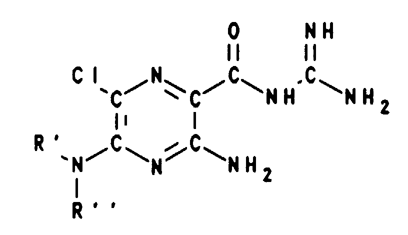
Amilorid: R',R'' = H
Dimethylamilorid: R',R'' = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff-Atom tragen. In der deutschen Patentanmeldung P 42 04 575.4 (HOE 92/F 034, entsprechend der kanadischen offengelegten Patentanmeldung Nr. 2 089 439, offengelegt am 16. August 1993) werden Benzoylguanidine vorgeschlagen, in welchen aber die Substituenten nicht die nach der vorliegenden Erfindung beanspruchten Bedeutungen haben.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den bekannten Verbindungen weisen die Verbindungen nach der Erfindung eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:

| | |
|---|---|
| MeOH | Methanol |
| DMF | N,N-Dimethylformamid |
| El | electron impact |
| DCI | Desorption-Chemical Ionisation |
| RT | Raumtemperatur |
| EE | Ethylacetat (EtOAc) |
| mp | Schmelzpunkt |
| HEP | n-Heptan |
| DME | Dimethoxyethan |
| ES | Elektronenspray |
| FAB | Fast Atom Bombardment |
| CH₂Cl₂ | Dichlormethan |
| THF | Tetrahydrofuran |
| eq. | Äquivalent |
| i. Vak. | im Vakuum |

### Experimenteller Teil

Allgemeine Vorschriften zur Darstellung von in 2-Position harnstoffsubstituierten Benzoesäuren aus Isatosäureanhydriden:
Variante 1 A: Aus Isatosäureanhydriden und einem silylierten Amin 1.0 eq. des Isatosäureanhydrids in CH₂Cl₂ (2 ml/mmol) wurden mit 1.1 eq. eines trimethylsilylierten Amins versetzt und bei passender Temperatur (RT bis Rückfluß) gerührt. Nach dem Entfernen des Lösungsmittels i.Vak., wurde mit Wasser versetzt (1.5 ml/mmol). Das auskristallisierende Benzoesäurederivat wurde abfiltriert, mit Wasser gewaschen und i. Vak. getrocknet.
Variante 1 B: Aus N-silylierten Isatosäureanhydriden und einem Amin Zu 1.0 eq. des Isatosäureanhydrids und 1.0 eq. Triethylamin in CH₂Cl₂ oder CHCl₃ (2 ml/mmol) wurden bei RT 1.05 eq. Trimethylsilylchlorid gegeben und eine passende Zeit gerührt. Anschließend gab man 1.0 eq. des Amins hinzu und rührte bei passender Temperatur (RT bis Rückfluß). Die Aufarbeitung erfolgte analog Variante 1 A.

Allgemeine Vorschriften zur Herstellung von Benzoyl-guanidinen (I)
Variante 2 A: aus Benzoesäuren (II, L=OH)
   1.0 eq. des Benzoesäurederivates der Formel II löst bzw. suspendiert man in wasserfreiem THF ( 5 ml/mmol ) und versetzt sodann mit 1.1 eq.
   Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck im Rotationsverdampfer ab, versetzt mit Wasser, stellt mit 2N HCI auf pH 6 bis 7 und filtriert das entsprechende Benzoylguanidin (Formel I) ab. Die so erhaltenen
   Benzoylguanidine können durch Behandeln mit wäßriger, methanolischer oder etherischer Salzsäure oder anderen pharmakologisch verträglichen Säuren in die entsprechenden Salze übergeführt werden.
Variante 2 B: aus Benzoesäure-alkylestern (II, L=O-Alkyl)
   1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck im Rotationsverdampfer abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
   (Salzbildung vergleiche Variante A)
   Allgemeine Vorschrift zur Darstellung von harnstoff- und thioharnstoffsubstituierten Benzoesäureestern aus Aminobenzoesäureestern und Iso- bzw. Isothio-cyanaten
Variante 3:
   1.0 eq. des Aminobenzoesäureesters wurden in Toluol (2 ml/mmol) und Pyridin (4 eq.) mit Iso- bzw. Isothio-cyanat versetzt und bei 80°C gerührt. Nach vollständiger Reaktion (1-6 h) wurde die abgekühlte Lösung mit Wasser versetzt und mit 2N Salzsäure sauer gestellt. Die organische Phase wurde abgetrennt, die wäßrige Phase zweimal mit EE ausgeschüttelt, die organischen Phasen getrocknet und i. Vak. eingeengt. Der verbleibende Feststoff wurde mit Ether gewaschen und i. Vak. getrocknet.

### Beispiel 1: 5-Chlor-2-(piperidino-carbonylamino)-benzoylguanidin

wurde gemäß Variante 1 A und 2 A aus 5-Chlor-isatosäureanhydrid und N-Trimethylsilyl-piperidin dargestellt.
(Benzoesäure-Zwischenprodukt: Farblose Kristalle, mp 165°C)
Farblose Kristalle, mp 151°C.

### Beispiel 2: 2-(Piperidino-carbonylamino)-benzoylguanidin

wurde gemäß Variante 1 A und 2 A aus Isatosäureanhydrid und N-Trimethylsilyl-piperidin dargestellt.
(Benzoesäure-Zwischenprodukt: Farblose Kristalle, mp 136°C)
Farblose Kristalle, mp 158°C.

### Beispiel 3: 5-Chlor-2-(dimethylamino-carbonylamino)-benzoylguanidinhydrochlorid

wurde gemäß Variante 1 A und 2 A aus 5-Chlor-isatosäureanhydrid und N,N-Dimethyl-N-trimethylsilyl-amin dargestellt.
(Benzoesäure-Zwischenprodukt: Farblose Kristalle, mp 177°C)
Farblose Kristalle, mp 174°C.

### Beispiel 4: 5-Chlor-2-(morpholino-carbonylamino)-benzoylguanidin-hydrochlorid

wurde gemäß Variante 1 A und 2 A aus 5-Chlor-isatosäureanhydrid und N-Trimethylsilyl-morpholin dargestellt.
(Benzoesäure-Zwischenprodukt: Farblose Kristalle, mp 187°C)
Farblose Kristalle, mp 162°C.

### Beispiel 5: 5-Chlor-2-(4-methyl-piperazin-1-yl-carbonylamino)-benzoylguanidindihydrochlorid

wurde gemäß Variante 1 B und 2 A aus 5-Chlor-isatosäureanhydrid und 4-N-Methyl piperazin dargestellt.
(Benzoesäure-Zwischenprodukt: Farblose Kristalle, mp 207°C)
Farblose Kristalle, mp 163°C.

### Beispiel 6: 5-Chlor-2-(pyrrolidin-1-yl-carbonylamino)-benzoylguanidinhydrochlorid

wurde gemäß Variante 1 A und 2 A aus 5-Chlor-isatosäureanhydrid und N-Trimethylsilyl-pyrrolidin dargestellt.
(Benzoesäure-Zwischenprodukt: Farblose Kristalle, mp 193°C)
Farblose Kristalle, mp 164°C.

### Beispiel 7: 5-Chlor-2-(tert.-butylamino-carbonylamino)-benzoylguanidinhydrochlorid

wurde gemäß Variante 1 A und 2 A aus 5-Chlor-isatosäureanhydrid und N-Trimethylsilyl-tert.-butylamin dargestellt.
(Benzoesäure-Zwischenprodukt: Farblose Kristalle, mp 155°C)
Farblose Kristalle, mp 145°C.

### Beispiel 8: 3-(n-Propylamino-carbonylamino)-benzoylguanidin-hydrochlorid

wurde gemäß Variante 3 und 2 B aus 3-Aminobenzoesäuremethylester und n-Propylisocyanat dargestellt.
(Benzoesäureester-Zwischenprodukt: Farblose Kristalle, mp 140°C)
Farblose Kristalle, mp 142°C.

### Beispiel 9: 3-(Phenylamino-carbonylamino)-benzoylguanidin-hydrochlorid

wurde gemäß Variante 3 und 2 B aus 3-Aminobezoesäuremethylester und Phenylisocyanat dargestellt.
(Benzoesäureester-Zwischenprodukt: Farblose Kristalle, mp 159°C)
Farblose Kristalle, mp 182°C.

### Beispiel 10: 3-(Cyclohexylamino-carbonylamino)-benzoylguanidin-hydrochlorid

wurde gemäß Variante 3 und 2 B aus 3-Aminobezoesäuremethylester und Cyclohexylisocyanat dargestellt.
(Benzoesäureester-Zwischenprodukt: Farblose Kristalle, mp 122°C)
Amorpher Feststoff.

### Beispiel 11: 3-(Ethylamino-thiocarbonylamino)-benzoylguanidin-hydrochlorid

wurde gemäß Variante 3 und 2 B aus 3-Aminobezoesäuremethylester und Ethylisothiocyanat dargestellt.
(Benzoesäureester-Zwischenprodukt: Farblose Kristalle, mp 130°C)
Farblose Kristalle, mp 130°C.

### Beispiel 12: 3-(Phenylamino-thiocarbonylamino)-benzoylguanidin-hydrochlorid

wurde gemäß Variante 3 und 2 B aus 3-Aminobezoesäuremethylester und Phenylisothiocyanat dargestellt.
(Benzoesäureester-Zwischenprodukt: Farblose Kristalle, mp 130°C)
Farblose Kristalle, mp 145°C.

### Beispiel 13: 3-(Cyclohexylamino-thiocarbonylamino)-benzoylguanidinhydrochlorid

wurde gemäß Variante 3 und 2 B aus 3-Aminobezoesäuremethylester und Cyclohexylisothiocyanat dargestellt.
(Benzoesäureester-Zwischenprodukt: Farblose Kristalle, mp 115°C)
Farblose Kristalle, mp 158°C.

### Beispiel 14: 4-Chlor-3-(n-propylamino-carbonylamino)-benzoylguanidinhydrochlorid

wurde gemäß Variante 3 und 2 B aus 4-Chlor-3-aminobezoesäuremethylester und
n-Propylisocyanat dargestellt.
(Benzoesäureester-Zwischenprodukt: Farblose Kristalle, mp 140°C)
Farblose Kristalle, mp 230°C.

### Beispiel 15: 4-Chlor-3-(phenylamino-carbonylamino)-benzoylguanidinhydrochlorid

wurde gemäß Variante 3 und 2 B aus 4-Chlor-3-aminobezoesäuremethylester und Phenylisocyanat dargestellt.
(Benzoesäureester-Zwischenprodukt: Farblose Kristalle, mp 186°C)
Farblose Kristalle, mp 232°C.

### Beispiel 16: 4-Chlor-3-(cyclohexylamino-carbonylamino)-benzoylguanidinhydrochlorid

wurde gemäß Variante 3 und 2 B aus 4-Chlor-3-aminobezoesäuremethylester und Cyclohexylisocyanat dargestellt.
(Benzoesäureester-Zwischenprodukt: Farblose Kristalle, mp 158°C)
Farblose Kristalle, mp 223°C.

### Beispiel 17: 4-Chlor-3-(ethylamino-thiocarbonylamino)-benzoylguanidinhydrochlorid

wurde gemäß Variante 3 und 2 B aus 4-Chlor-3-aminobezoesäuremethylester und Ethylisothiocyanat dargestellt.
(Benzoesäureester-Zwischenprodukt: Farblose Kristalle, mp 152°C)
Farblose Kristalle, mp 145°C.

### Beispiel 18: 4-Chlor-3-(phenylamino-thiocarbonylamino)-benzoylguanidinhydrochlorid

wurde gemäß Variante 3 und 2 B aus 4-Chlor-3-aminobezoesäuremethylester und Phenylisothiocyanat dargestellt.
(Benzoesäureester-Zwischenprodukt: Farblose Kristalle, mp 130°C)
Farblose Kristalle, mp 145°C.

### Beispiel 19: 4-Chlor-3-(cyclohexylamino-thiocarbonylamino)-benzoylguanidinhydrochlorid

wurde gemäß Variante 3 und 2 B aus 4-Chlor-3-aminobezoesäuremethylester und Cyclohexylisothiocyanat dargestellt.
Farblose Kristalle, mp 190°C.

### Beispiel 20: 3,5-Dichlor-4-(n-propylamino-carbonylamino)-benzoylguanidinhydrochlorid

wurde gemäß Variante 3 und 2 B aus 3,5-Dichlor-4-aminobezoesäuremethylester und n-Propylisocyanat dargestellt.
(Benzoesäureester-Zwischenprodukt: Farbloses Öl)
Farblose Kristalle, mp 220°C.

### Beispiel 21: 3,5-Dichlor-4-(phenylamino-carbonylamino)-benzoylguanidinhydrochlorid

wurde gemäß Variante 3 und 2 B aus 3,5-Dichlor-4-aminobenzoesäuremethylester und Phenylisocyanat dargestellt.
(Benzoesäureester-Zwischenprodukt: Farblose Kristalle, mp 82°C)
Farblose Kristalle, mp 234°C.

### Pharmakologische Daten:

### Inhibition des Na⁺/H ⁺-Exchangers von Kaninchenerythozyten:

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-1-hydroxymethylaminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.
Der Na⁺-Nettoinflux wurde aus der Differenz zwischen Natrium- Ausgangswerten und dem Natriumgehalt der Erythrozyten nach Inkubation errechnet. Der Amilorid-hemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers:

| Beispiel | IC₅₀ (µmol) |
|---|---|
| 1 | 1 - 2 |
| 3 | 3 |
| 7 | 3 - 8 |
| 17 | 3 - 5 |
| 20 | 2 - 5 |
| 21 | 1 |

## Patentansprüche

1. Benzoylguanidine der Formel I 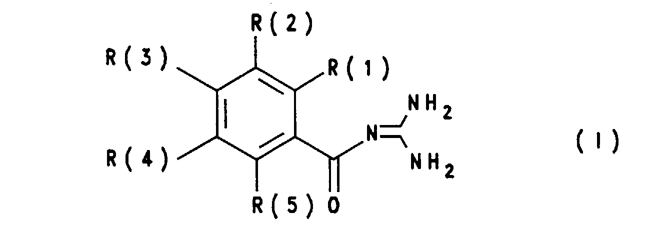 worin bedeuten:
R(1), R(3) oder R(4) -NR(6) C=X NR(7)R(8),
X Sauerstoff, S,
R(6) Wasserstoff, (C₁-C₈)-Alkyl, (C₁-C₈)-Perfluoralkyl, (C₃-C₈)-Alkenyl, -CₙH₂ₙ-R(9),
n Null, 1, 2, 3 oder 4,
R(9) (C₃-C₈)-Cycloalkyl, Phenyl, Biphenylyl oder Naphthyl, wobei die Aromaten nicht substituiert oder substituiert sind mit 1 - 3 Substituenten aus der Gruppe F, Cl, CF₃, Methyl, Methoxy oder NR(10)R(11),
R(10) und R(11) H, (C₁-C₄)-Alkyl oder (C₁-C₄)-Perfluoralkyl;
R(7) Wasserstoff, (C₁-C₈)-Alkyl, (C₁-C₈)-Perfluoralkyl, (C₃-C₈)-Alkenyl, -CₒH₂ₒ-R(12),
o Null, 1, 2, 3 oder 4,
R(12) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(13)R(14),
R(13) und R(14) H, (C₁-C₄)-Alkyl oder (C₁-C₄)-Perfluoralkyl;
R(8) definiert wie R(7);
wobei R(7) und R(8) auch gemeinsam 4 oder 5 Methylengruppen sein können, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5) unabhängig voneinander
Wasserstoff, F, Cl, Br, I, -Oₜₐ(C₁-C₈)-Alkyl, -O_{tb}(C₃-C₈)-Alkenyl, -O_{tc}(CH₂)_{b}C_{d}F_{2d+1}, -O_{td}CₚH₂ₚR(18),
oder bis zu 2 Gruppen CN, NO₂, NR(16)R(17),
b Null oder 1,
d 1, 2, 3, 4, 5, 6 oder 7,
ta Null oder 1,
tb Null oder 1,
tc Null oder 1,
td Null oder 1,
p Null, 1, 2, 3 oder 4,
R(18) (C₃-C₈)-Cycloalkyl, Phenyl, Biphenylyl oder Naphthyl, wobei die Aromaten nicht substituiert oder substituiert sind mit 1 - 3 Substituenten aus der Gruppe F, Cl, CF₃, Methyl, Methoxy oder NR(19)R(20),
R(19) und R(20) Wasserstoff, (C₁-C₄)-Alkyl, (C₁C₄)-Perfluoralkyl,
R(16) Wasserstoff, (C₁-C₈)-Alkyl, (C₁-C₈)-Perfluoralkyl, (C₃-C₈)-Alkenyl, -C_{q}H_{2q}-R(21),
q Null, 1, 2, 3 oder 4,
R(21) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(22)R(23),
R(22) und R(23) Wasserstoff, (C₁-C₄)-Alkyl oder (C₁-C₄)-Perfluoralkyl,
R(17) Wasserstoff, (C₁-C₈)-Alkyl, (C₁-C₈)-Perfluoralkyl, (C₅-C₈)-Alkenyl, -CᵣH₂ᵣ-R(24),
r Null, 1, 2, 3 oder 4,
R(24) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy oder NR(25)R(26),
R(25) und R(26) Wasserstoff, (C₁-C₄)-Alkyl oder (C₁-C₄)-Perfluoralkyl;
wobei R(16) und R(17) auch gemeinsam 4 oder 5 Methylengruppen sein können, von denen eine CH₂-Gruppe durch Sauerstoff, S, NH, N-CH₃ oder N-Benzyl ersetzt sein kann,
sowie deren pharmazeutisch verträgliche Salze.

2. Verbindungen der Formel I nach Anspruch 1, in der bedeuten:
R(1), R(3) oder R(4) -NR(6) C=X NR(7)R(8),
X Sauerstoff, S,
R(6) Wasserstoff, (C₁-C₈)-Alkyl, (C₁-C₈)-Perfluoralkyl, (C₃-C₈)-Alkenyl, -CₙH₂ₙ-R(9),
n Null oder 1,
R(9) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(10)R(11),
R(10) und R(11) H, CH₃,
R(7) Wasserstoff, (C₁-C₈)-Alkyl, (C₁-C₈)-Perfluoralkyl, (C₃-C₈)-Alkenyl, -CₒH₂ₒ-R(12),
o Null oder 1,
R(12) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(13)R(14),
R(13) und R(14) H, CH₃,
R(8) definiert wie R(7),
wobei R(7) und R(8) auch gemeinsam 4 oder 5 Methylengruppen sein können, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5) sind unabhängig voneinander Wasserstoff, F, Cl, Br, I, (C₁-C₈)-Alkyl, (C₃-C₈)-Alkenyl,
(C₁-C₇)-Perfluoralkyl, CₚH₂ₚR(18),
oder bis zu 2 Gruppen CN, NO₂, NR(16)R(17),
p Null oder 1,
R(18) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(19)R(20),
R(19) und R(20) Wasserstoff, CH₃,
R(16) Wasserstoff, (C₁-C₈)-Alkyl, (C₁-C₈)-Perfluoralkyl, (C₃-C₈)-Alkenyl, -C_{q}H_{2q}-R(21),
q Null oder 1,
R(21) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(22)R(23),
R(22) und R(23) Wasserstoff, CH₃,
R(17) Wasserstoff, (C₁-C₈)-Alkyl, (C₁-C₈)-Perfluoralkyl, (C₃-C₈)-Alkenyl, -CᵣH₂ᵣ-R(24),
r Null oder 1,
R(24) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(25)R(26),
R(25) und R(26) Wasserstoff oder CH₃,
wobei R(16) und R(17) auch gemeinsam 4 oder 5 Methylengruppen sein können, von denen eine CH₂-Gruppe durch Sauerstoff, S, NH, N-CH₃ oder N-Benzyl ersetzt sein kann,
sowie deren pharmazeutisch verträgliche Salze.

3. Verbindungen der Formel I nach Anspruch 1, in der bedeuten:
R(1), R(3) oder R(4) -NR(6) C =X NR(7)R(8),
X Sauerstoff, S,
R(6) Wasserstoff, (C₁-C₄)-Alkyl, (C₁-C₄)-Perfluoralkyl,
R(7) Wasserstoff, (C₁-C₄)-Alkyl, (C₁-C₄)-Perfluoralkyl, (C₃-C₈)-Alkenyl, -CₒH₂ₒ-R(12),
o Null oder 1,
R(12) (C₃-C₈)-Cycloalkyl, Phenyl,
das nicht substituiert oder substituiert sind mit 1 - 3 Substituenten aus der Gruppe F, Cl, CF₃, Methyl, Methoxy oder NR(13)R(14),
R(13) und R(14) H, CH₃,
R(8) definiert wie R(7),
wobei R(7) und R(8) auch gemeinsam 4 oder 5 Methylengruppen sein können, von denen eine CH₂-Gruppe durch Sauerstoff, S, NH, N-CH₃ oder N-Benzyl ersetzt sein kann;
die jeweils verbleibenden Substituenten R(2), R(3), R(4), R(5) oder R(1), R(2), R(4), R(5) oder R(1), R(2), R(3), R(5) sind unabhängig voneinander
Wasserstoff, F, Cl, Br, I, (C₁-C₄)-Alkyl, (C₁-C₄)-Perfluoralkyl, CₚH₂ₚR(18), oder bis zu 2 Gruppen CN, NO₂, NR(16)R(17),
p Null oder 1,
R(18) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(19)R(20),
R(19) und R(20) Wasserstoff, CH₃,
R(16) Wasserstoff, (C₁-C₄)-Alkyl, (C₁-C₄)-Perfluoralkyl,
R(17)
Wasserstoff, (C₁-C₄)-Alkyl, (C₁-C₄)-Perfluoralkyl, (C₃-C₈)-Alkenyl, -CᵣH₂ᵣ-R(24),
r Null oder 1,
R(24) (C₃-C₈)-Cycloalkyl, Phenyl,
das nicht substituiert oder substituiert ist mit 1 - 3 Substituenten aus der Gruppe F, Cl, CF₃, Methyl, Methoxy oder NR(25)R(26),
R(25) und R(26) Wasserstoff oder CH₃,
wobei R(16) und R(17) auch gemeinsam 4 oder 5 Methylengruppen sein können, von denen eine CH₂-Gruppe durch Sauerstoff, S, NH, N-CH₃ oder N-Benzyl ersetzt sein kann,
sowie deren pharmazeutisch verträgliche Salze.

4. Verfahren zur Herstellung einer Verbindung I nach Anspruch 1, dadurch gekennzeichnet, daß man eine Verbindung der Formel II 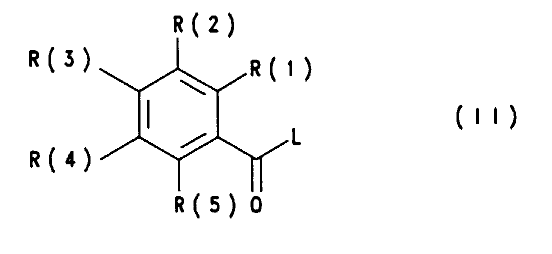 mit Guanidin umsetzt, worin R(1) bis R(5) die oben angegebene Bedeutung besitzen und L für eine leicht nucleophil substituierbare leaving group steht.

5. Verwendung einer Verbindung I nach Anspruch 1 zur Herstellung eines Medikaments zu Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch 1 zur Herstellung eines Medikaments zur Behandlung von Krankheiten, bei denen die Zellproliferation eine primäre oder sekundäre Ursache darstellt.

15. Heilmittel gekennzeichnet durch eine wirksame Menge einer Verbindung I nach Anspruch 1.

## Claims

1. A benzoylguanidine of the formula I 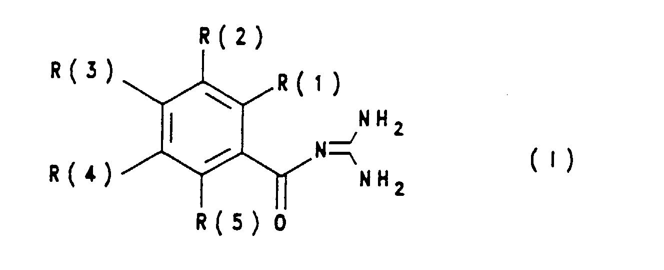 in which:
R(1), R(3) or R(4) is -NR(6) C = X NR(7)R(8),
X is oxygen, S,
R(6) is hydrogen, (C₁-C₈)-alkyl, (C₁-C₈)-perfluoroalkyl, (C₃-C₈)-alkenyl, -CₙH₂ₙ-R(9),
n is zero, 1, 2, 3 or 4,
R(9) is (C₃-C₈)-cycloalkyl, phenyl, biphenylyl or naphthyl,
where the aromatic systems are not substituted or are substituted by 1-3 substituents from the group consisting of F, Cl, CF₃, methyl, methoxy or NR(10)R(11),
R(10) and R(11) are H, (C₁-C₄)-alkyl or (C₁-C₄)-perfluoroalkyl;
R(7) is hydrogen, (C₁-C₈)-alkyl, (C₁-C₈)-perfluoroalkyl, (C₃-C₈)-alkenyl, -CₒH₂ₒ-R(12),
o is zero, 1, 2, 3 or 4,
R(12) is (C₃-C₈)-cycloalkyl, phenyl, biphenylyl or naphthyl,
where the aromatic systems are not substituted or are substituted by 1-3 substituents from the group consisting of F, Cl, CF₃, methyl, methoxy or NR(13)R(14),
R(13) and R(14) are H, (C₁-C₄)-alkyl or (C₁-C₄)-perfluoroalkyl;
R(8) is defined as R(7);
where R(7) and R(8) can also together be 4 or 5 methylene groups, of which one CH₂ group can be replaced by oxygen, S, NH, N-CH₃ or N-benzyl;
the remaining substituents in each case R(2), R(3), R(4), R(5) or R(1), R(2), R(4), R(5) or R(1), R(2), R(3), R(5) independently of one another
are hydrogen, F, Cl, Br, I, -Oₜₐ(C₁-C₈)-alkyl, -O_{tb}(C₃-C₈)-alkenyl, -O_{tc}(CH₂)_{b}C_{d}F_{2d+1}, -O_{td}CₚH₂ₚR(18), or up to 2 groups CN, NO₂, NR(16)R(17),
b is zero or 1,
d is 1, 2, 3, 4, 5, 6 or 7,
ta is zero or 1,
tb is zero or 1,
tc is zero or 1,
td is zero or 1,
p is zero, 1, 2, 3 or 4,
R(18) is (C₃-C₈)-cycloalkyl, phenyl, biphenylyl or naphthyl, where the aromatic systems are not substituted or are substituted by 1-3 substituents from the group consisting of F, Cl, CF₃, methyl, methoxy or NR(19)R(20),
R(19) and R(20) are hydrogen, (C₁-C₄)-alkyl, (C₁-C₄)-perfluoroalkyl,
R(16) is hydrogen, (C₁-C₈)-alkyl, (C₁-C₈)-perfluoroalkyl, (C₃-C₈)-alkenyl, (C_{q}H_{2q}-R(21),
q is zero, 1, 2, 3 or 4,
R(21) is (C₃-C₈)-cycloalkyl, phenyl, biphenylyl or naphthyl,
where the aromatic systems are not substituted or are substituted by 1-3 substituents from the group consisting of F, Cl, CF₃, methyl, methoxy or NR(22)R(23),
R(22) and R(23) are hydrogen, (C₁-C₄)-alkyl or (C₁-C4)-perfluoroalkyl,
R(17) is hydrogen, (C₁-C₈)-alkyl, (C₁-C₈)-perfluoroalkyl, (C₃-C₈)-alkenyl, -CᵣH₂ᵣ-R(24),
r is zero, 1, 2, 3 or 4,
R(24) is (C₃-C₈)-cycloalkyl, phenyl, biphenylyl or naphthyl,
where the aromatic systems are not substituted or are substituted by 1-3 substituents from the group consisting of F, Cl, CF₃, methyl, methoxy or NR(25)R(26),
R(25) and R(26) are hydrogen, (C₁-C₄)-alkyl or (C₁-C₄)-perfluoroalkyl,
where R(16) and R(17) can also together be 4 or 5 methylene groups, of which one CH₂ group can be replaced by oxygen, S, NH, N-CH₃ or N-benzyl;
and their pharmaceutically tolerable salts.

2. A compound of the formula I as claimed in claim 1, in which:
R(1), R(3) or R(4) is -NR(6) C = X NR(7)R(8),
X is oxygen, S,
R(6) is hydrogen, (C₁-C₈)-alkyl, (C₁-C₈)-perfluoroalkyl, (C₃-C₈)-alkenyl, -CₙH₂ₙ-R(9),
n is zero or 1,
R(9) is (C₃-C₈)-cycloalkyl, phenyl,
which is not substituted or is substituted by 1-3 substituents from the group consisting of F, Cl, CF₃, methyl, methoxy or NR(10)R(11),
R(10) and R(11) are H, CH₃,
R(7) is hydrogen, (C₁-C₈)-alkyl, (C₁-C₈)-perfluoroalkyl, (C₃-C₈)-alkenyl, -CₒH₂ₒ-R(12),
o is zero or 1,
R(12) is (C₃-C₈)-cycloalkyl, phenyl,
where the aromatic systems are not substituted or are substituted by 1-3 substituents from the group consisting of F, Cl, CF₃, methyl, methoxy or NR(13)R(14),
R(13) and R(14) are H, CH₃,
R(8) is defined as R(7);
where R(7) and R(8) can also together be 4 or 5 methylene groups, of which one CH₂ group can be replaced by oxygen, S, NH, N-CH₃ or N-benzyl;
the remaining substituents in each case R(2), R(3), R(4), R(5) or R(1), R(2), R(4), R(5) or R(1), R(2), R(3), R(5) independently of one another
are hydrogen, F, Cl, Br, I, (C₁-C₈)-alkyl, (C₃-C₈)-alkenyl, (C₁-C₇)-perfluoroalkyl,CₚH₂ₚR(18), or up to 2 groups CN, NO₂, NR(16)R(17),
p is zero or 1,
R(18) is (C₃-C₈)-cycloalkyl, phenyl,
which is not substituted or is substituted by 1-3 substituents from the group consisting of F, Cl, CF₃, methyl, methoxy or NR(19)R(20),
R(19) and R(20) are hydrogen, CH₃,
R(16) is hydrogen, (C₁-C₈)-alkyl, (C₁-C₈)-perfluoroalkyl, (C₃-C₈)-alkenyl, -C_{q}H_{2q}-R(21),
q is zero or 1,
R(21) is (C₃-C₈)-cycloalkyl, phenyl,
which is not substituted or is substituted by 1-3 substituents from the group consisting of F, Cl, CF₃, methyl, methoxy or NR(22)R(23),
R(22) and R(23) are hydrogen, CH₃,
R(17) is hydrogen, (C₁-C₈)-alkyl, (C₁-C₈)-perfluoroalkyl, (C₃-C₈)-alkenyl, -CᵣH₂ᵣ-R(24),
r is zero or 1,
R(24) is (C₃-C₈)-cycloalkyl, phenyl,
which is not substituted or is substituted by 1-3 substituents from the group consisting of F, Cl, CF₃, methyl, methoxy or NR(25)R(26),
R(25) and R(26) are hydrogen or CH₃,
where R(16) and R(17) can also together be 4 or 5 methylene groups, of which one CH₂ group can be replaced by oxygen, S, NH, N-CH₃ or N-benzyl,
and their pharmaceutically tolerable salts.

3. A compound of the formula I as claimed in claim 1, in which:
R(1), R(3) or R(4) is -NR(6) C = X NR(7)R(8),
X is oxygen, S,
R(6) is hydrogen, (C₁-C₄)-alkyl, (C₁-C₄)-perfluoroalkyl,
R(7) is hydrogen, (C₁-C₄)-alkyl, (C₁-C₄)-perfluoroalkyl, (C₃-C₈)-alkenyl, -CₒH₂ₒ-R(12),
o is zero or 1,
R(12) is (C₃-C₈)-cycloalkyl, phenyl,
which is not substituted or is substituted by 1-3 substituents from the group consisting of F, Cl, CF₃, methyl, methoxy or NR(13)R(14),
R(13) and R(14) are H, CH₃,
R(8) is defined as R(7),
where R(7) and R(8) can also together be 4 or 5 methylene groups, of which one CH₂ group can be replaced by oxygen, S, NH, N-CH₃ or N-benzyl;
the remaining substituents in each case R(2), R(3), R(4), R(5) or R(1), R(2), R(4), R(5) or R(1), R(2), R(3), R(5) independently of one another
are hydrogen, F, Cl, Br, I, (C₁-C₄)-alkyl, (C₁-C₄)-perfluoroalkyl, CₚH₂ₚR(18),
or up to 2 groups CN, NO₂, NR(16)R(17),
p is zero or 1,
R(18) is (C₃-C₈)-cycloalkyl, phenyl,
which is not substituted or is substituted by 1-3 substituents from the group consisting of F, Cl, CF₃, methyl, methoxy or NR(19)R(20),
R(19) and R(20) are hydrogen, CH₃,
R(16) is hydrogen, (C₁-C₄)-alkyl, (C₁-C₄)-perfluoroalkyl,
R(17) is hydrogen, (C₁-C₄)-alkyl, (C₁-C₄)-perfluoroalkyl, (C₃-C₈)-alkenyl, -CᵣH₂ᵣ-R(24),
r is zero or 1,
R(24) is (C₃-C₈)-cycloalkyl, phenyl,
which is not substituted or is substituted by 1-3 substituents from the group consisting of F, Cl, CF₃, methyl, methoxy or NR(25)R(26),
R(25) and R(26) are hydrogen or CH₃,
where R(16) and R(17) can also together be 4 or 5 methylene groups, of which one CH₂ group can be replaced by oxygen, S, NH, N-CH₃ or N-benzyl,
and their pharmaceutically tolerable salts.

4. A process for the preparation of a compound as claimed in claim 1, which comprises reacting a compound of the formula II 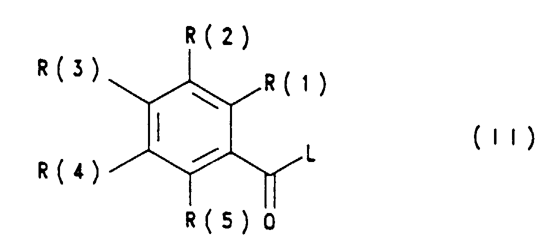 in which R(1) to R(5) have the meaning indicated above and L represents an easily nucleophilically substitutable leaving group, with guanidine.

5. The use of a compound I as claimed in claim 1 for the production of a medicament for the treatment of arrhythmias.

6. The use of a compound I as claimed in claim 1 for the production of a medicament for the treatment or prophylaxis of cardiac infarct.

7. The use of a compound I as claimed in claim 1 for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim 1 for the production of a medicament for the treatment or prophylaxis of ischemic conditions of the peripheral and central nervous system and of stroke.

10. The use of a compound I as claimed in claim 1 for the production of a medicament for the treatment or prophylaxis of ischemic conditions of the peripheral organs and members.

11. The use of a compound I as claimed in claim 1 for the production of a medicament for the treatment or states of shock.

12. The use of a compound I as claimed in claim 1 for the production of a medicament for use in surgical operations and organ transplantation.

13. The use of a compound I as claimed in claim 1 for the production of a medicament for the preservation and storage of transplants for surgical measures.

14. The use of a compound I as claimed in claim 1 for the production of a medicament for the treatment of diseases in which cell proliferation is a primary or secondary cause.

15. A medicine which comprises an effective amount of a compound I as claimed in claim 1.

## Revendications

1. Benzoylguanidines de formule I 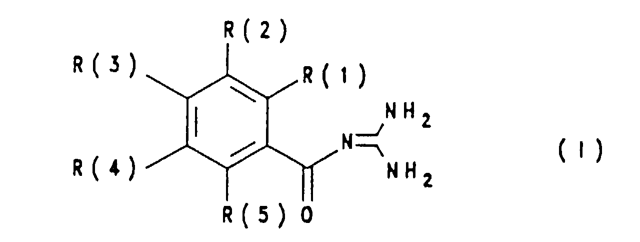 dans laquelle
R(1), R(3) ou R(4) représente -NR(6)C=XNR(7)R(8),
X représente un atome d'oxygène ou de soufre,
R(6) représente un atome d'hydrogène ou un groupe alkyle en C₁-C₈, perfluoroalkyle en C₁-C₈, alcényle en C₃-C₈, -CₙH₂ₙ-R(9),
n est égal à zéro, 1, 2, 3 ou 4,
R(9) représente un groupe cycloalkyle en C₃-C₈, phényle, biphénylyle ou naphtyle, les radicaux aromatiques n'étant pas substitués ou portant 1 à 3 substituants choisis dans l'ensemble constitué par F, Cl, CF₃, les groupes méthyle, méthoxy et NR(10)R(11),
R(10) et R(11) représentant H ou un groupe alkyle en C₁-C₄ ou perfluoroalkyle en C₁-C₄;
R(7) représente un atome d'hydrogène ou un groupe alkyle en C₁-C₈, perfluoroalkyle en C₁-C₈, alcényle en C₃-C₈, -CₒH₂ₒ-R(12),
o est égal à zéro, 1, 2, 3 ou 4,
R(12) représente un groupe cycloalkyle en C₃-C₈, phényle, biphénylyle ou naphtyle,
les radicaux aromatiques n'étant pas substitués ou portant 1 à 3 substituants choisis dans l'ensemble constitué par F, Cl, CF₃, les groupes méthyle, méthoxy et NR(13)R(14),
R(13) et R(14) représentant H ou un groupe alkyle en C₁-C₄ ou perfluoroalkyle en C₁-C₄;
R(8) est défini comme R(7);
R(7) et R(8) pouvant également être ensemble 4 ou 5 groupes méthylène, parmi lesquels un groupe CH₂ peut être remplacé par un atome d'oxygène ou de soufre ou par NH, N-CH₃ ou par le groupe N-benzyle;
les substituants R(2), R(3), R(4), R(5) ou R(1), R(2), R(4), R(5) ou R(1), R(2), R(3), R(5), restant dans chaque cas représentent, indépendamment les uns des autres,
un atome d'hydrogène ou de F, Cl, Br, I, ou un groupe -Oₜₐ-alkyle en C₁-C₈, -O_{tb}-alcényle en C₃-C₈, -O_{tc}(CH₂)_{b}C_{d}F_{2d+1}, -O_{td}CₚH₂ₚR(18),
ou jusqu'à 2 groupes CN, NO₂, NR(16)R(17),
b étant zéro ou 1,
d étant 1, 2, 3, 4, 5, 6 ou 7,
ta étant zéro ou 1,
tb étant zéro ou 1,
tc étant zéro ou 1,
td étant zéro ou 1,
p étant zéro, 1, 2, 3 ou 4,
R(18) représentant un groupe cycloalkyle en C₃-C₈, phényle, biphénylyle ou naphtyle,
les radicaux aromatiques n'étant pas substitués ou portant 1 à 3 substituants choisis dans l'ensemble constitué par F, Cl, CF₃, les groupes méthyle, méthoxy et NR(19)R(20),
R(19) et R(20) représentant un atome d'hydrogène ou un groupe alkyle en C₁-C₄ ou perfluoroalkyle en C₁-C₄,
R(16) représentant un atome d'hydrogène ou un groupe alkyle en C₁-C₈, perfluoroalkyle en C₁-C₈, alcényle en C₃-C₈, C_{q}H_{2q}R(21),
q est égal à zéro, 1, 2, 3 ou 4,
R(21) représente un groupe cycloalkyle en C₃-C₈, phényle, biphénylyle ou naphtyle,
les radicaux aromatiques n'étant pas substitués ou portant 1 à 3 substituants choisis dans l'ensemble constitué par F, Cl, CF₃, les groupes méthyle, méthoxy et NR(22)R(23),
R(22) et R(23) représentant un atome d'hydrogène ou un groupe alkyle en C₁-C₄ ou perfluoroalkyle en C₁-C₄,
R(17) représentant un atome d'hydrogène ou un groupe alkyle en C₁-C₈, perfluoroalkyle en C₁-C₈, alcényle en C₃-C₈, CᵣH₂ᵣR(24),
r est égal à zéro, 1, 2, 3 ou 4,
R(24) représente un groupe cycloalkyle en C₃-C₈, phényle, biphénylyle ou naphtyle,
les radicaux aromatiques n'étant pas substitués ou portant 1 à 3 substituants choisis dans l'ensemble constitué par F, Cl, CF₃, les groupes méthyle, méthoxy et NR(25)R(26),
R(25) et R(26) représentant un atome d'hydrogène ou un groupe alkyle en C₁-C₄ ou perfluoroalkyle en C₁-C₄,
R(16) et R(17) pouvant également être ensemble 4 ou 5 groupes méthylène, parmi lesquels un groupe CH₂ peut être remplacé par un atome d'oxygène ou de soufre ou par NH, N-CH₃ ou par le groupe N-benzyle,
et leurs sels pharmaceutiquement acceptables.

2. Composés de formule I selon la revendication 1, dans lesquels
R(1), R(3) ou R(4) représente-NR(6)C=XNR(7)R(8),
X représente un atome d'oxygène ou de soufre,
R(6) représente un atome d'hydrogène ou un groupe alkyle en C₁-C₈, perfluoroalkyle en C₁-C₈, alcényle en C₃-C₈, CₙH₂ₙR(9),
n est égal à zéro ou 1,
R(9) représente un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(10) R(11),
R(10) et R(11) représentant H ou CH₃;
R(7) représente un atome d'hydrogène ou un groupe alkyle en C₁-C₈, perfluoroalkyle en C₁-C₈, alcényle en C₃-C₈, CₒH₂ₒ-R(12),
o est égal à zéro ou 1,
R(12) représente un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(13)R(14),
R(13) et R(14) représentant H ou CH₃;
R(8) est défini comme R(7);
R(7) et R(8) pouvant également être ensemble 4 ou 5 groupes méthylène, parmi lesquels un groupe CH₂ peut être remplacé par un atome d'oxygène ou de soufre ou par NH, N-CH₃ ou par le groupe N-benzyle;
les substituants R(2), R(3), R(4), R(5) ou R(1), R(2), R(4), R(5) ou R(1), R(2), R(3), R(5), restant dans chaque cas représentent, indépendamment les uns des autres,
un atome d'hydrogène ou de F, Cl, Br, I, ou un groupe alkyle en C₁-C₈, alcényle en C₃-C₈, perfluoroalkyle en C₁-C_{7,} CₚH₂ₚR(18),
ou jusqu'à 2 groupes CN, NO₂, NR(16)R(17),
p étant zéro ou 1,
R(18) représentant un groupe cycloalkyle en C₃-C₈, phényle
qui n'est pas substitué ou porte 1 à 3 substituants choisis dans l'ensemble constitué par F, Cl, CF₃, les groupes méthyle, méthoxy ou NR(19)R(20),
R(19) et R(20) représentant un atome d'hydrogène ou CH₃
R(16) représentant un atome d'hydrogène ou un groupe alkyle en C₁-C₈, perfluoroalkyle en C₁-C₈, alcényle en C₃-C₈, -C_{q}H_{2q}-R(21),
q est égal à zéro ou 1,
R(21) représente un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(22)R(23),
R(22) et R(23) représentant un atome d'hydrogène ou CH₃,
R(17) représentant un atome d'hydrogène ou un groupe alkyle en C₁-C₈, perfluoroalkyle en C₁-C₈, alcényle en C₃-C₈, -CᵣH₂ᵣ-R(24),
r est égal à zéro ou 1,
R(24) représente un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(25)R(26),
R(25) et R(26) représentant un atome d'hydrogène ou CH₃,
R(16) et R(17) pouvant également être ensemble 4 ou 5 groupes méthylène, parmi lesquels un groupe CH₂ peut être remplacé par un atome d'oxygène ou de soufre ou par NH, N-CH₃ ou par le groupe N-benzyle,
et leurs sels pharmaceutiquement acceptables.

3. Composés de formule I selon la revendication 1, dans lesquels
R(1), R(3) ou R(4) représente -NR(6)C=XNR(7)R(8),
X représente un atome d'oxygène ou de soufre,
R(6) représente un atome d'hydrogène ou un groupe alkyle en C₁-C₄, perfluoroalkyle en C₁-C₄,
R(7) représente un atome d'hydrogène ou un groupe alkyle en C₁-C₄, perfluoroalkyle en C₁-C₄, alcényle en C₃-C₈, CₒH₂ₒ-R(12),
o est égal à zéro ou 1,
R(12) représente un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(13)R(14),
R(13) et R(14) représentant H ou CH₃;
R(8) est défini comme R(7);
R(7) et R(8) pouvant également être ensemble 4 ou 5 groupes méthylène, parmi lesquels un groupe CH₂ peut être remplacé par un atome d'oxygène ou de soufre ou par NH, N-CH₃ ou par le groupe N-benzyle;
les substituants R(2), R(3), R(4), R(5) ou R(1), R(2), R(4), R(5) ou R(1), R(2), R(3), R(5), restant dans chaque cas représentent, indépendamment les uns des autres,
un atome d'hydrogène ou de F, Cl, Br, I, ou un groupe alkyle en C₁-C₄, perfluoroalkyle en C₁-C₄, CₚH₂ₚR(18),
ou jusqu'à 2 groupes CN, NO₂, NR(16)R(17),
p étant zéro ou 1,
R(18) représentant un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(19)R(20),
R(19) et R(20) représentant un atome d'hydrogène ou CH₃
R(16) représentant un atome d'hydrogène ou un groupe alkyle en C₁-C₄, perfluoroalkyle en C₁-C₄,
R(17) représentant un atome d'hydrogène ou un groupe alkyle en C₁-C₄, perfluoroalkyle en C₁-C₄, alcényle en C₃-C₈, CᵣH₂ᵣR(24),
r est égal à zéro ou 1,
R(24) représente un groupe cycloalkyle en C₃-C₈, phényle
qui n'est pas substitué ou porte 1 à 3 substituants choisis dans l'ensemble constitué par F, Cl, CF₃, les groupes méthyle, méthoxy et NR(25)R(26),
R(25) et R(26) représentant un atome d'hydrogène ou CH₃,
R(16) et R(17) pouvant également être ensemble 4 ou 5 groupes méthylène, parmi lesquels un groupe CH₂ peut être remplacé par un atome d'oxygène ou de soufre ou par NH, N-CH₃ ou par le groupe N-benzyle.
et leurs sels pharmaceutiquement acceptables.

4. Procédé pour la préparation d'un composé I selon la revendication 1, caractérisé en ce que l'on fait réagir avec de la guanidine un composé de formule II 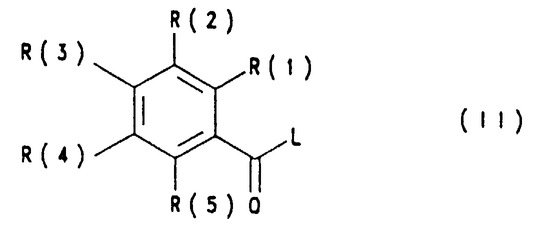 dans laquelle R(1) à R(5) ont les significations données plus haut et L représente un groupe partant pouvant être aisément remplacé par substitution nucléophile.

5. Utilisation d'un composé I selon la revendication 1, pour la fabrication d'un médicament destiné au traitement d'arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Médicament caractérisé par une teneur efficace en un composé de formule I selon la revendication 1.
